# EUROPEAN PATENT APPLICATION

(11) **EP 2 052 703 A1**
(43) Date of publication of application: **29.04.2009**
(21) Application number: 08253403.3
(22) Date of filing: 21.10.2008
(51) Int. Cl.: A61F 13/15

(54) **Sanitary article assembly including an overwrap for disposal of a soiled sanitary article**

(30) Priority: 22.10.2007 US 981580 P; 17.12.2007 US 957853
(71) Applicant: JOHNSON & JOHNSON INC., Montreal, Quebec H1N 2G4 (CA)
(72) Inventor: Amiot, Suzanne, Repentigny, Quebec J5Y 3M7 (CA); Brisebois, France, Lachenaie, Quebec J6W 5N2 (CA); Hann, Dragana, Anjou, Quebec H1J 2B6 (CA)
(74) Representative: Kirsch, Susan Edith

(57) **Abstract**

The present invention relates to sanitary absorbent articles and in particular to a sanitary absorbent article assembly including an overwrap structure for packaging a clean sanitary article, the overwrap structure including a pocket for receiving a soiled sanitary article for disposal.

## Description

### CROSS REFERENCE TO RELATED APPLICATIONS

This application claims priority to Application No. 60/981,580 filed on October 22, 2007, the entire contents of which are incorporated by reference herein.

### FIELD OF THE INVENTION

The present invention generally relates to sanitary absorbent articles and in particular to a sanitary absorbent article assembly including an overwrap structure for packaging a clean sanitary article, the overwrap structure also including a pocket for receiving a soiled sanitary article for disposal.

### BACKGROUND OF THE INVENTION

Conventional sanitary absorbent articles such as sanitary napkins and the like are individually packaged prior to use to maintain the sanitary napkin clean prior to use. Often each individual sanitary napkin is folded in an overlapping configuration, commonly referred to as "tri-folded", and individually packaged in a polypropylene or polyethylene film pouch, commonly referred to as the "overwrap". In this manner, each individual napkin is maintained in a hygienic condition prior to use. When the user is ready to use the napkin, the napkin is removed from the "overwrap", unfolded, and arranged in the undergarment for use. A problem with above described overwrap structure is that once the napkin has been used (i.e. soiled) there is no effective way for the user to dispose of the soiled absorbent article in a hygienic manner. Rather, the user is often forced to simply place the soiled absorbent article in a refuse container or in the alternative the user may first wrap the article in toilet paper or the like prior to disposal.

In view of the foregoing, the inventors have recognized a need to provide a sanitary napkin assembly including an overwrap that effectively protects the sanitary napkin in a hygienic manner prior to use and also provides means to easily dispose of a soiled sanitary napkin in a hygienic manner.

### SUMMARY OF THE INVENTION

In view of the foregoing, the present invention provides a sanitary article assembly including a sanitary article having a tri-folded configuration prior to use, an overwrap for packaging the tri-folded sanitary article prior to use, the overwrap having a first wall and a second wall arranged in opposed relationship to one another, an outer surface and an inner surface, a first end portion, a second end portion and an intermediate portion arranged between the first and second end portions, an inner surface, an outer surface, and a lip having a first position in which it is arranged in overlapping relationship to the inner surface, the overwrap having a first closed configuration wherein the first and second end portions of the overwrap are arranged in overlapping relationship to thereby define a pouch for receiving the tri-folded sanitary article prior to use, the overwrap having a second open configuration wherein the first and second walls of the overwrap define a pocket for receiving a soiled sanitary article, the lip being structured and arranged to be manually inverted by a user, when the overwrap is in the second configuration, from the first position to a second position wherein the lip is arranged in overlapping relationship with the outer surface of the overwrap to thereby close the pocket.

The present invention provides, according to a second aspect of the invention, A method of packaging, using and disposing of a sanitary article including the steps of providing a sanitary article having a trifolded configuration prior to use, the sanitary article having a garment facing surface and an adhesive arranged on the garment facing surface for securing the sanitary article to an undergarment during use, the tri-folded configuration defining a first end portion of the sanitary article, a second end portion of the sanitary article and an intermediate portion arranged between the first end portion and the second end portion, packaging the trifolded sanitary article in an overwrap for protecting the sanitary article prior to use, the overwrap having a first wall and a second wall arranged in opposed relationship to one another, an outer surface and an inner surface, a first end portion, a second end portion and an intermediate portion arranged between the first and second end portions, an inner surface, an outer surface, and a lip having a first position in which it is arranged in overlapping relationship to the inner surface, the overwrap having a first closed configuration wherein the first and second end portions of the overwrap are arranged in overlapping relationship to thereby define a pouch for receiving the tri-folded sanitary article prior to use, overwrap having a second open configuration wherein the first and second walls of the overwrap define a pocket for receiving a soiled sanitary article, removing the sanitary article from the overwrap by manually arranging the overwrap in the second open configuration, removing a soiled sanitary article from an undergarment, inserting the soiled sanitary article into the pocket, manually inverting the lip when the overwrap is in the second configuration, from the first position to a second position wherein the lip is arranged in overlapping relationship with the outer surface of the overwrap to thereby close the pocket.

The present invention provides, according to a third aspect of the invention, a sanitary article assembly including a sanitary article, an overwrap for packaging the tri-folded sanitary article prior to use, the overwrap having a first wall and a second wall arranged in opposed relationship to one another to define, and a lip having a first open configuration, the overwrap having a first closed configuration for holding an unused sanitary article, the overwrap having a second open configuration wherein the first and second walls of the overwrap define a pocket for receiving a soiled sanitary article, and the lip being structured and arranged to be manually inverted by a user, when the overwrap is in the second configuration, from the first position to a second closed position to thereby close the pocket.

### BRIEF DESCRIPTION OF THE DRAWINGS

Examples of embodiments of the present invention will now be described with reference to the drawings, in which:
Fig. 1 is a perspective view of an overwrap according to the present invention in a closed configuration, the overwrap containing an unused sanitary absorbent article;
Fig. 2 is a perspective view of the overwrap of Fig. 1 in a partially open configuration;
Fig. 3 is a perspective view of the overwrap of Fig. 1 in an open configuration;
Fig. 4 is a schematic view showing the manner of attachment of the sanitary absorbent article according to the invention to an undergarment;
Fig. 5 is a schematic view of the overwrap of Fig. 1 depicting the manner in which the overwrap may receive a soiled sanitary absorbent article;
Fig. 6 is schematic view of the overwrap of Fig. 1 containing a soiled sanitary absorbent article in a partially rolled configuration; and
Fig. 7 is a schematic view of the overwrap of Fig. 1 containing a soiled sanitary absorbent article in a fully rolled configuration.

### DETAILED DESCRIPTION OF THE INVENTION

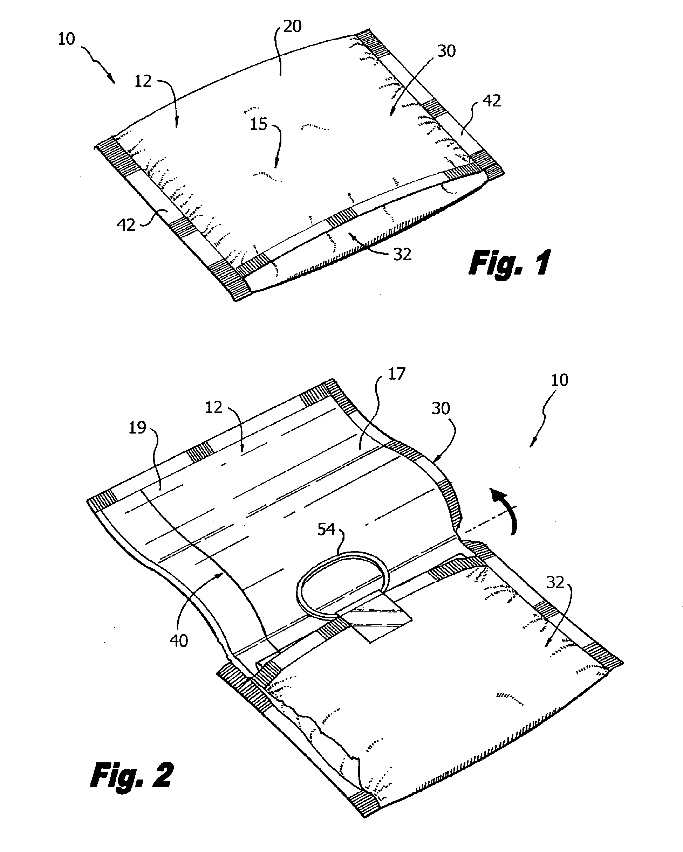

Referring to Figs. 1-7 the sanitary napkin assembly 10 according to the present invention generally includes an overwrap 12 and a sanitary napkin 14. Although the invention will be described herein with reference to a sanitary napkin 14 the invention may be utilized with other disposable sanitary absorbent articles such as adult incontinence products, panty liners, diapers and the like.

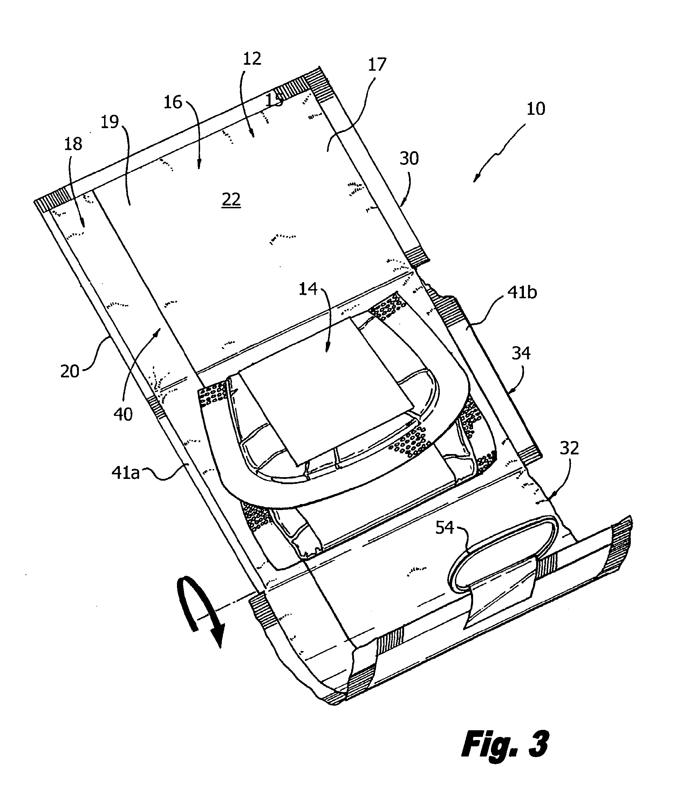

As best seen in Fig. 1 and Fig. 3 the sanitary napkin 14, prior to use, is arranged in a trifolded configuration and contained within the overwrap 12. The overwrap 12 is shown in a closed configuration in Fig. 1 with the sanitary napkin 14 contained within the overwrap 12. The overwrap 12 is also arranged in a tri-folded configuration so as to define a pouch 15 for receiving the napkin 14 prior to use. The overwrap 12 is preferably constructed from a liquid impermeable polymeric film such as polyethylene or polypropylene, although other suitable liquid impermeable materials may also be employed.

The closed configuration of the overwrap shown in Fig. 1 is the configuration in which the user would find the overwrap 12 and sanitary napkin 14 prior to use. Fig. 3 shows the overwrap 12 in an open configuration enabling the user to manually remove the sanitary napkin 14 from the overwrap 12 for use. It is noted that overwrap 12 may be easily manually opened from the configuration shown in Fig. 1 to the configuration shown in Fig. 3.

Referring to Fig. 3, the overwrap 12 includes a first wall 16 and a second wall 18 which are arranged in opposed relationship to one another. An outer surface 20 of the second wall 18 defines an outer surface of the overwrap 12 when the overwrap is in the closed configuration, as shown in Fig. 1. The first wall 16 includes a primary portion 17 and a lip portion 19. The first wall 16 primarily defines the inner surface 22 of the overwrap 12, as shown in Fig. 3. The lip portion 19 is arranged, in a first position, such that it is in overlapping relationship to primary portion 17 and more particularly to the inner surface 22 of the overwrap 12.

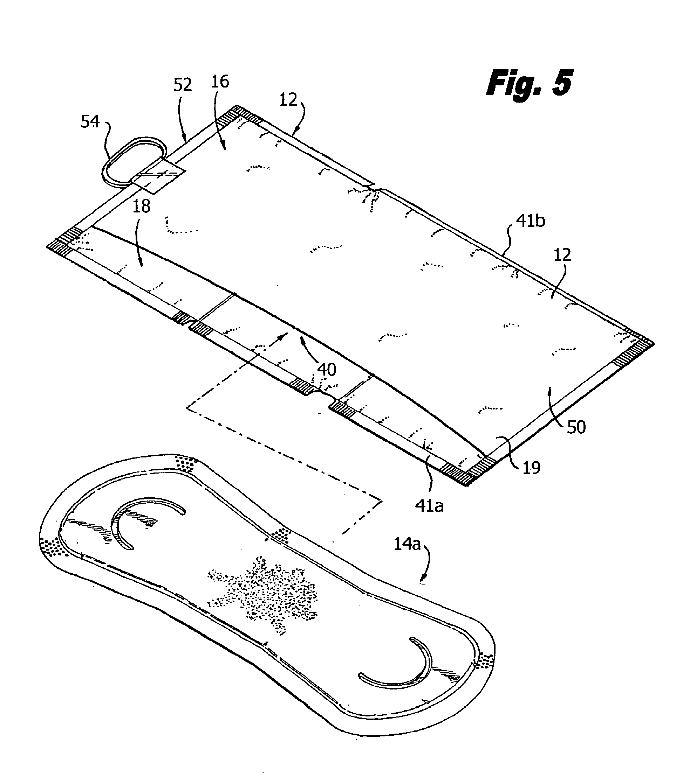

As best seen in Fig. 5, the first wall 16 and second wall 18 are arranged in opposed relationship to one another such that they define a pocket 40 therebetween for receiving a soiled sanitary napkin 14a. Although the first wall 16 is depicted in the Figures as being arranged in opposed relationship to the inner surface of the second wall 18 it could also be arranged in opposed relationship to the outer surface 20 of the second wall 18. That is, the pocket 40 could also be arranged on the outer surface 20 of the overwrap 12.

The first wall 16 preferably extends substantially from a first transverse edge 41a of the overwrap 12 to a second transverse edge 41b of the overwrap 12 such that the pocket 40 extends substantially from the first transverse edge 41a to the second transverse edge 41b. In another embodiment of the invention, the first wall 16 may extend the entire length from the first transverse edge 41a to the second transverse edge 41b such that the pocket 40 extends the entire length from the first transverse edge 41a to the second transverse edge 41 of the overwrap 12.

As shown in Fig. 3, the overwrap 12 generally includes a first end portion or tri-section 30, a second end portion or tri-section 32 and an intermediate portion or tri-section 34. As best seen in Fig. 1, when the overwrap 12 is in the closed configuration prior to use the first end portion 30 is arranged in overlapping relationship with the second end portion 32. In the closed configuration, the first end portion 30, second end portion 32 and intermediate portion 34 are preferably secured to one another along their respective edges by means of a crimp seal 42. In this manner, the overwrap 12 is maintained in the closed configuration shown in Fig. 1 to maintain the sanitary napkin 14 in a hygienic state until the user is ready to use the sanitary napkin 14.

Once a user is ready to the use the napkin 14 the user may manually open the overwrap 12 from the closed configuration shown in Fig. 1 to the open configuration shown in Fig. 3 by unfolding the first end portion 30 and second end portion 32 of the overwrap from their overlapping condition shown in Fig. 1 to thereby break the crimp seals 42 and place the overwrap 12 in the open configuration shown in Fig. 3. Once the overwrap 12 is placed in the open configuration shown in Fig. 3 the user may access the napkin 14 for use.

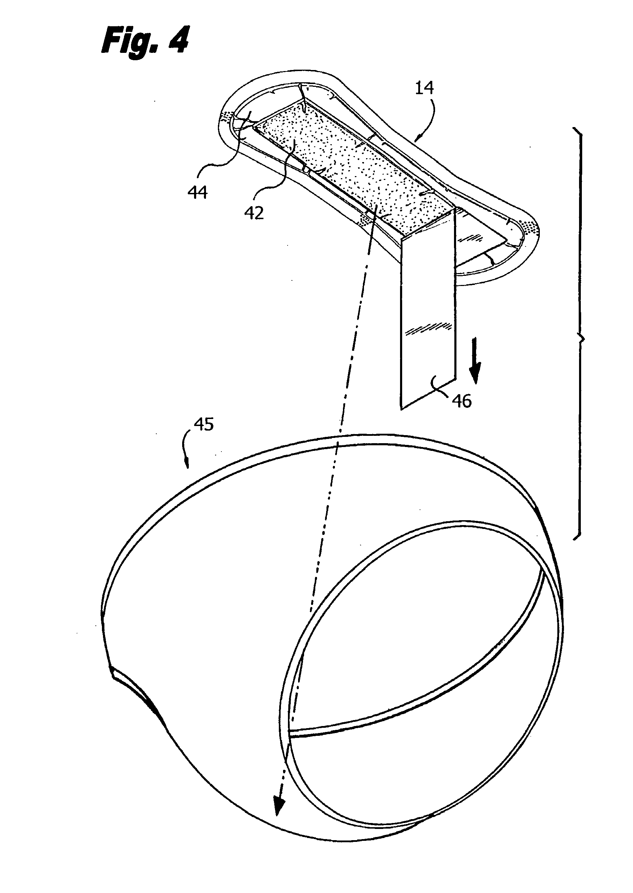

As shown in Fig. 3, the sanitary napkin 14 is arranged in a tri-folded configuration in the overwrap 12 prior to use. In one preferred embodiment of the invention shown in Fig. 4, the sanitary napkin 14 is provided with a garment attachment adhesive 42 arranged on a garment facing surface 44 of the napkin 14 which functions to securely attach the sanitary napkin 14 to an undergarment 45 during use. The adhesive 42 is preferably covered by a removable release paper 46 to protect the garment attachment adhesive 42 prior to use of the napkin 14. In another embodiment of the invention, not shown in the Figures, the removable release paper 46 may be omitted. However, in such an embodiment, the inner surface 22 of the overwrap 12 should be provided with a nonstick surface coating, e.g. silicone, to facilitate the removal of the napkin 14 from the overwrap 12.

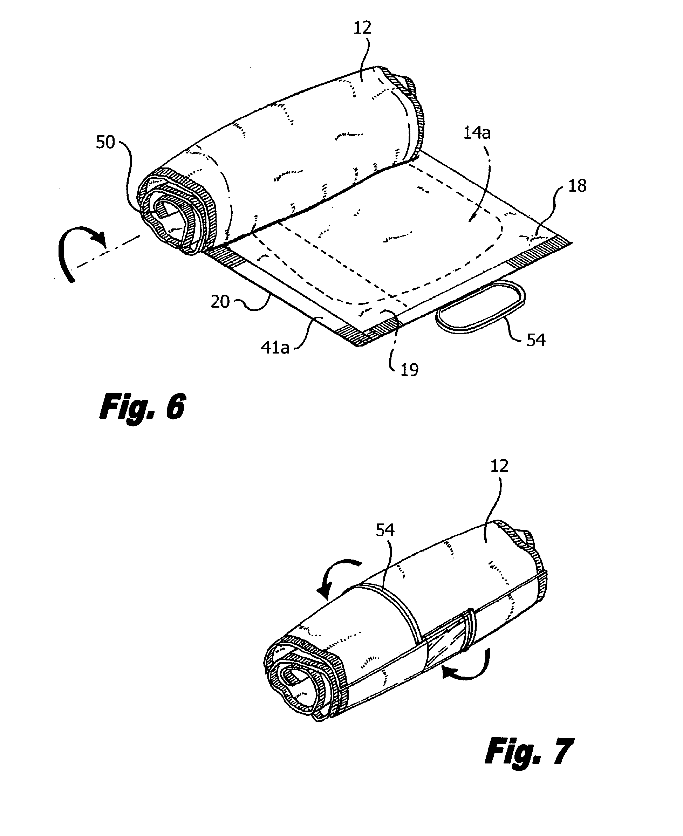

After a user has removed the napkin 14 from the overwrap 12 and arranged the napkin in the user's undergarment, the user may place a soiled napkin 14a within the pocket 40 defined between the first 16 and second 18 walls of the overwrap 12, as shown in Fig. 5. After placing the soiled napkin 14a in the pocket 40 the user may then invert the lip portion 19 of the overwrap 12 from the first position shown in Fig. 5 wherein the lip portion 19 is arranged in overlapping relationship to the inner surface 22 of the overwrap 12 to a second position shown in Fig. 6 wherein the lip portion 19 is arranged in overlapping relationship to the outer surface 20 of the overwrap 12. By inverting the lip portion 19 from the position shown in Fig. 5 to the position shown in Fig. 6 the lip portion 19 functions to close the pocket 40 by passing over the first transverse edge 41a of the overwrap thereby closing the opening to the pocket 40. As shown in Fig. 6, after the user has placed the soiled napkin 14a in the pocket 40 and inverted the lip portion 19 as described above, the user may then roll the overwrap from a first end 50 of overwrap 12 to a second end 52 of the overwrap 12. Secured to the second end 52 of the overwrap is a device 54 for selectively maintaining the overwrap 12 in a rolled configuration. In one embodiment of the invention the device 54 may comprise a piece of elastomeric material such as rubber or the like that can be wrapped around the overwrap 12 when the overwrap 12 is in a rolled configuration, as shown in Fig. 7. Other devices may also be employed provided that they function to maintain the overwrap 12 in the rolled configuration shown in Fig. 7.

A method of using the absorbent article assembly 10 according to the present invention will now be described with reference to Figures 1-7. As shown in Figs. 1 and 3 the napkin 14 is provided in a trifolded configuration prior to use and packaged within the overwrap 12. When the user desires to access the napkin 14 the user manually opens the overwrap 12 from the first closed configuration shown in Fig. 1 to a second open configuration shown in Fig. 3. The user may then remove the napkin 14 from the overwrap 12 for use. As shown in Fig. 5, the user may then insert a soiled sanitary napkin 14a into the pocket 40 defined between the first wall 16 and second wall 18 of the overwrap 12. After placing the soiled sanitary napkin 14a into the pocket 40, the user may then invert the lip portion 19 of the overwrap 12 from the position shown in Fig. 5 to the position shown in Fig. 6 to thereby close the pocket 40. Thereafter, the user may roll the overwrap 12 from the first end 50 of the overwrap to a second end 52 of the overwrap 12 as shown in Fig. 6. Thereafter, the user may maintain the overwrap in the rolled configuration shown in Fig. 6 by manually activating the device 54 for selectively maintaining the overwrap 12 in a rolled configuration. In particular the user may wrap the device 54 around the rolled overwrap 12 to thereby maintain the overwrap 12 in the rolled configuration, as shown in Fig. 7. Thereafter, the user may easily dispose of the soiled napkin 14a.

Variations of the sanitary article assembly according to the present invention may be apparent to those of skill in the art based upon the disclosure of the present application. Thus, it is intended that the present application cover the modifications and variations of this invention provided that they come within the scope of the appended claims and their equivalents.

## Claims

1. A sanitary article assembly comprising:
a sanitary article having a tri-folded configuration prior to use;
an overwrap for packaging the tri-folded sanitary article prior to use, the overwrap having a first wall and a second wall arranged in opposed relationship to one another, an outer surface and an inner surface, a first end portion, a second end portion and an intermediate portion arranged between the first and second end portions, an inner surface, an outer surface, and a lip having a first position in which it is arranged in overlapping relationship to the inner surface;
the overwrap having a first closed configuration wherein the first and second end portions of the overwrap are arranged in overlapping relationship to thereby define a pouch for receiving the tri-folded sanitary article prior to use;
the overwrap having a second open configuration wherein the first and second walls of the overwrap define a pocket for receiving a soiled sanitary article; and
the lip being structured and arranged to be manually inverted by a user, when the overwrap is in the second configuration, from the first position to a second position wherein the lip is arranged in overlapping relationship with the outer surface of the overwrap to thereby close the pocket.

2. The sanitary article assembly according to claim 1, wherein an opening of the pocket extends from a first transverse edge seam of the overwrap to a second transverse edge seam of the overwrap.

3. The sanitary article assembly according to claim 1 or claim 2, wherein the pocket is structured and arranged to receive the soiled napkin in an unfolded flat configuration.

4. The sanitary article according to any preceding claim, wherein the overwrap is structured and arranged to be manually rolled from the second configuration to a third rolled configuration when the soiled sanitary article is received in the pocket.

5. The sanitary article according to claim 4, wherein the overwrap further includes a device for selectively maintaining the overwrap in the third rolled configuration.

6. The sanitary article according to claim 5, wherein the device for selectively maintaining the overwrap in the third rolled configuration comprises an elastomeric member adapted to encircle overwrap in the third rolled configuration.

7. The sanitary article according to claim 5, wherein the device for selectively maintaining the overwrap in the third rolled configuration comprises an adhesive tab adapted to be selective adhered to an outer surface of the overwrap.

8. A method of packaging, using and disposing of a sanitary article comprising the steps of:
providing a sanitary article having a trifolded configuration prior to use, the sanitary article having a garment facing surface and an adhesive arranged on the garment facing surface for securing the sanitary article to an undergarment during use, the tri-folded configuration defining a first end portion of the sanitary article, a second end portion of the sanitary article and an intermediate portion arranged between the first end portion and the second end portion;
packaging the trifolded sanitary article in an overwrap for protecting the
sanitary article prior to use, the overwrap having a first wall and a second wall arranged in opposed relationship to one another, an outer surface and an inner surface, a first end portion, a second end portion and an intermediate portion arranged between the first and second end portions, an inner surface, an outer surface, and a lip having a first position in which it is arranged in overlapping relationship to the inner surface, the overwrap having a first closed configuration wherein the first and second end portions of the overwrap are arranged in overlapping relationship to thereby define a pouch for receiving the tri-folded sanitary article prior to use, overwrap having a second open configuration wherein the first and second walls of the overwrap define a pocket for receiving a soiled sanitary article;
removing the sanitary article from the overwrap by manually arranging the overwrap in the second open configuration;
removing a soiled sanitary article from an undergarment;
inserting the soiled sanitary article into the pocket;
manually inverting the lip when the overwrap is in the second configuration, from the first position to a second position wherein the lip is arranged in overlapping relationship with the outer surface of the overwrap to thereby close the pocket.

9. The method according to claim 8, wherein an opening of the pocket extends from a first transverse edge seam of the overwrap to a second transverse edge seam of the overwrap.

10. The method according to claim 8 or claim 9, wherein the pocket is structured and arranged to receive the soiled napkin in an unfolded flat configuration.

11. The method according to any one of claims 8 to 10, further comprising manually rolling the overwrap from the second configuration to a third rolled configuration after the soiled sanitary article is received in the pocket.

12. The method according to claim 11, further comprising maintaining the overwrap in the third rolled configuration by manually activating a device for maintaining the overwrap in the third rolled configuration.

13. The method according to claim 12, wherein the device an elastomeric member adapted to encircle overwrap in the third rolled configuration.

14. The method according to claim 12, wherein the device comprises an adhesive tab adapted to be selective adhered to an outer surface of the overwrap.

15. A sanitary article assembly comprising:
a sanitary article;
an overwrap for packaging the tri-folded sanitary article prior to use, the overwrap having a first wall and a second wall arranged in opposed relationship to one another to define, and a lip having a first open configuration;
the overwrap having a first closed configuration for holding an unused sanitary article;
the overwrap having a second open configuration wherein the first and second walls of the overwrap define a pocket for receiving a soiled sanitary article; and
the lip being structured and arranged to be manually inverted by a user, when the overwrap is in the second configuration, from the first position to a second closed position to thereby close the pocket.
